Europäisches Patentamt

European Patent Office

Office européen des brevets

(19)

(11) Veröffentlichungsnummer: **0 162 320**
**A1**

(12) # EUROPÄISCHE PATENTANMELDUNG

(21) Anmeldenummer: **85104978.3**

(22) Anmeldetag: **24.04.85**

(51) Int. Cl.⁴: **A 61 K 31/135**
**A 61 K 31/70**
**//(A61K31/70, 31:135)**

(30) Priorität: **26.04.84 DE 3415575**

(43) Veröffentlichungstag der Anmeldung:
**27.11.85 Patentblatt 85/48**

(84) Benannte Vertragsstaaten:
**AT BE CH DE FR GB IT LI LU NL SE**

(71) Anmelder: **Klinge Pharma GmbH**
**Berg-am-Laim-Strasse 129**
**D-8000 München 80(DE)**

(72) Erfinder: **Niederhauser, Alois**
**Feilenreit 7**
**D-8221 Neukirchen am Teisenberg(DE)**

(72) Erfinder: **Seibel, Klaus**
**Haberlstrasse 9**
**D-8032 Gräfelfing(DE)**

(74) Vertreter: **Wuesthoff, Franz, Dr.-Ing. et al,**
**Patentanwälte Wuesthoff -v. Pechmann-Behrens-Goetz**
**Schweigerstrasse 2**
**D-8000 München 90(DE)**

(54) **Verwendung von Etilefrin und Etilefrinpivalat zur Herstellung eines Arzneimittels für die Langzeitbehandlung nicht hypotoniebedingter Durchblutungsstörungen.**

(57) Etilefrin oder Etilefrinpivalat eignet sich zur Langzeitbehandlung nicht hypotoniebedingter Durchblutungsstörungen, wie z.B. peripherer arterieller Stenosen und führt überraschenderweise zu einem kontinuierlichen stabilen Anstieg des systolischen Blutdrucks und der Blutdruckamplitude und einem verbesserten Herzschlagvolumen, ohne den diastolischen Blutdruck oder die Herzfrequenz zu beeinflussen. Verstärkt wird die pharmazeutische Wirkung noch, wenn das Etilefrin in Kombination mit einem Roßkastaniensamenextrakt bei der Langzeitbehandlung verwendet wird.

EP 0 162 320 A1

0162320

KLINGE PHARMA GmbH
München
EP-59 048

B e s c h r e i b u n g :

Verwendung von Etilefrin und Etilefrinpivalat zur Herstellung eines Arzneimittels für die Langzeitbehandlung
nicht hypotoniebedingter Durchblutungsstörungen.

Die Erfindung bezieht sich auf die Verwendung von Etilefrin und Etilefrinpivalat sowie auf die Verwendung einer Kombination von Etilefrin und Rosskastaniensamen-Extrakt bei der Behandlung von Durchblutungsstörungen, z.B. infolge Arteriosklerose und erniedrigtem Herz-Schlagvolumen im Alter.

Das Sympathomimetikum Etilefrin wurde bisher als schnell wirkendes Mittel bei Kreislaufstörungen, insbesondere bei Hypotonie und der orthostatischen Fehlregulation verwendet, und ist in Standardwerken der angewandten Pharmakologie ausschliesslich als Mittel zur Steigerung des Blutdrucks beschrieben. Der Blutdruckanstieg wird durch die bekannte vasokonstriktorische Wirkung der Substanz bewirkt. Etilefrin übt neben seiner vasokonstriktorischen Wirkung auch eine stimulierende Wirkung auf das Herz aus, die sich in einem jedoch sehr geringen Anstieg des Herzschlagvolumens äussert. Bei peroraler oder parenteraler Verabreichung solcher Sympathomimetika wird neben einem Blutdruckanstieg am Ruhe- kreislauf gleichzeitig eine unerwünschte Frequenzbeschleuni- gung am Herzen beobachtet, insbesondere, wenn man versucht durch Gabe höherer Wirkstoffdosen einen relevanten Anstieg des Herzschlagvolumens zu erzielen.

0162320

Aufgrund der kurzen Halbwertlebensdauer von 2,2 Stunden für die Eliminierung des Etilefrins aus dem Blutstrom kann auch bei Gabe höherer Dosen keine über längere Zeit stabile, erhöhte Blutdruckamplitude beim Patienten erreicht werden, obwohl dies eine Forderung bei der langfristigen Beseitigung von Durchblutungsstörungen und ihren Symptomen ist. Wenn die Durchblutungsstörungen auf eine cerebrale oder periphere arterielle Stenose zurückzuführen ist, z.B. infolge Arteriosklerose, oder auf eine cerebrale Vasokonstriktion wie bei Migräne, dann ist die Applikation von Etilefrin aufgrund seiner vasokonstriktorischen Wirkung unangebracht, da eine weitere Minderung der peripheren Gefäßweite zu einer weiteren Verschlechterung der Blutzirkulation führen muß. Auch wurde festgestellt, dass bei üblicherweise verabreichten Dosen von 30 mg in Lösung per os bereits unangenehme Nebenwirkungen mit unerwünschter Vasokonstriktion auftreten können.

Es wurde nun überraschenderweise festgestellt, dass bei längerer regelmäßiger Verwendung geringer Dosen von Etilefrin und Etilefrinpivalat das Herzschlagvolumen deutlich und konstant gesteigert wird.

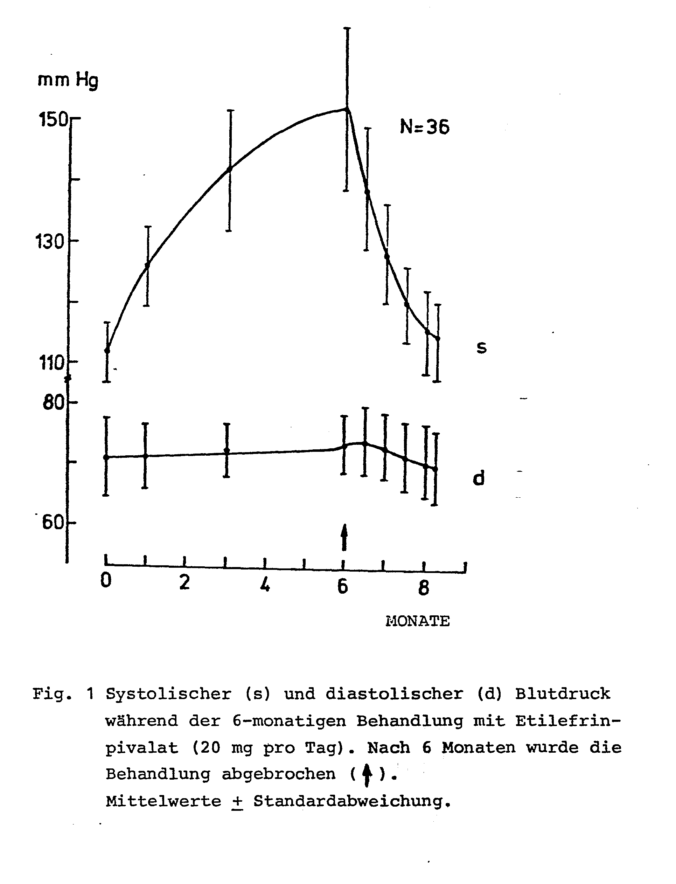

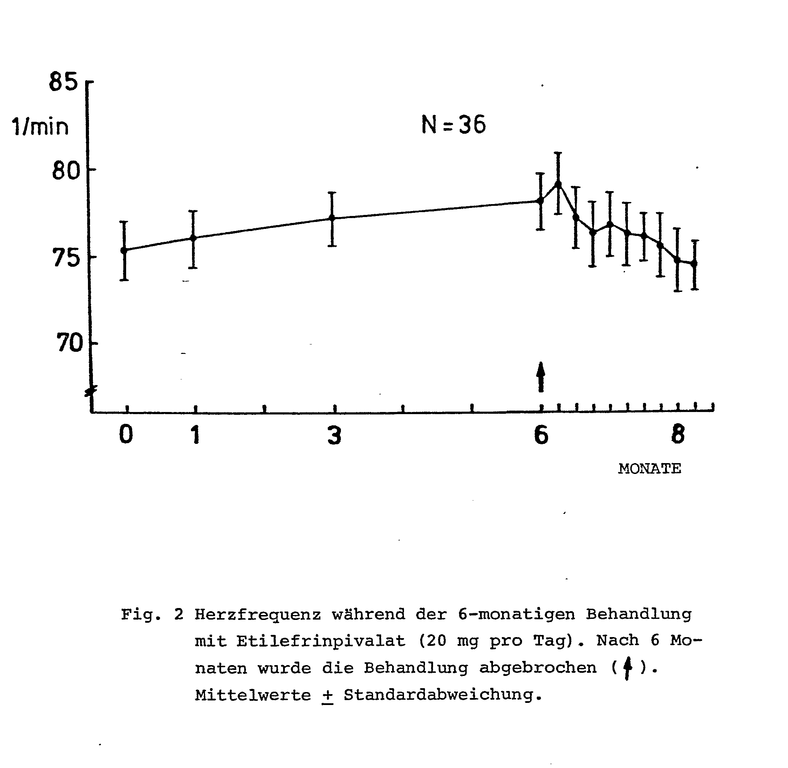

Bei einer Langzeitbehandlung von 6 Monaten und einer Dosierung von 20 mg Etilefrinpivalat pro Tag, was einer Verabreichung von 14,4 mg Etilefrin entspricht, wurde der systolische Blutdruck und die Blutdruckamplitude kontinuierlich verstärkt, während der diastolische Blutdurck und die Herzfrequenz nahezu gleich blieben (Fig.1 und 2). Besonders bemerkenswert ist hierbei, dass die Blutdruckamplitude während der Behandlungsphase von 6 Monaten ständig zunahm, obwohl aufgrund der niedrigen Halbwertslebensdauer des Etilefrins von 2,2 Stunden an eine Kummulation des Wirkstoffs im Blut nicht zu denken ist. In diesem Zusammenhang sei darauf hingewiesen, dass nach Verabreichung des Etilefrinpivalats nur Etilefrin im Blut gefunden wird, was bedeutet, dass das Etilefrinpivalat vollständig in Etilefrin und Pivalinsäure während der Absorption gespalten wird. Die Er-

0162320

höhung der Blutdruckamplitude und des systolischen Blutdrucks sind im Hinblick auf das therapeutische Ziel, das
Herzschlagvolumen im Falle einer cerebralen und peripheren
arteriellen Stenose oder Vasokonstriktion zu erhöhen, besonders hervorzuheben. Wie der gleichbleibende diastolische
Blutdruck zeigt, tritt während der Behandlungsphase bei der
gewählten Dosierung trotz der vasokonstriktorischen Wirkung
von Etilefrin keine Erhöhung des peripheren Widerstands auf,
so dass überraschenderweise bei der Behandlung von nichthypotoniebedingten Durchblutungsstörungen keine unerwünschte Vasokonstriktion auftritt.

Entsprechend der Ergebnisse wurden bei einer Langzeitbehandlung mit Etilefrin und Etilefrinpivalat für eine Vergleichsstudie erhalten. Bei der Langzeitbehandlung von
4 Wochen und einer Dosierung von 2 mal täglich 10,82 pro
mg Etilefrin (Handelsname Effortil$^{(R)}$) und 15 mg Etilefrinpivalat, was einer Verabreichung von je $5 \times 10^{-5}$ Mol, wurde
der systolische Blutdruck und die Blutdruckamplitude kontinuierlich verstärkt, während der diastolische Blutdruck
und die Herzfrequenz nahezu gleich blieben. Nach Beendigung der Behandlung mit Etilefrinpivalat war die Blutdruckamplitude im Stehen höher als im Liegen vor der Behandlung
und beinahe so hoch nach Behandlung mit Etilefrin.

Auch wenn der Wirkungsmechanismus bei der erfindungsgemässen Behandlung noch nicht aufgeklärt ist, ergibt sich ein
evidenter Vorteil bei der Behandlung oben genannter Durchblutungsstörungen, da die Tageszeit der Arzneimitteleinnahme bei einer Langzeitbehandlung von geringer Bedeutung
für die Zunahme des Herzschlagvolumens ist. Der therapeutische
Schutz des Patienten ist nicht nur während der Nacht gegeben, sondern auch am Morgen, wenn die Neigung zur orthostatischen Fehlregulation am grössten ist. Beachtenswert ist dabei noch, dass der Effekt der beiden Substanzen
auf die Blutdruckamplitude von der Körperlage des Patienten unabhängig ist, d.h. im Liegen und Stehen gleichermassen vorhanden ist.

/4

Durch Verwendung von Etilefrin bzw. Etilefrinpivalat ist somit zum ersten Mal die Möglichkeit gegeben, langfristig eine dauerhafte Steigerung des Herzschlagvolumens zu erreichen, um damit arterielle Stenosen, Durchblutungsstörungen der Netzhaut, cerebrovaskuläre Insuffiziens und auch das Altersherz, das ein erniedrigtes Schlagvolumen aufweist, zu behandeln, ohne dabei das Risiko einer unerwünschten Vasokonstriktion und einer erhöhten Herzfrequenz eingehen zu müssen.

Überraschenderweise hat sich auch gezeigt, dass die Wirkung des Etilefrins noch verstärkt werden kann, wenn bei der Behandlung neben dem Etilefrin gleichzeitig auch Rosskastaniensamenextrakt appliziert wird. Wie die Untersuchungen mit einem Kombinationspräparat gezeigt haben, wird dabei die Blutdruckamplitude noch weiter erhöht. Auch in diesem Fall ist die Blutdruckamplitude am Ende der 4. Behandlungswoche bei Verabreichung des Kombinationspräparates im Stehen ebenso gross, wie vor Beginn der Behandlung im Liegen.

Im folgenden wird die Erfindung anhand einer während der Behandlung durchgeführten Studie näher erläutert.

Zur Objektivierung der Wirkung der applizierten Substanzen wurde ein bis zwei Tage vor Behandlungsbeginn sowie am siebten, vierzehnten und achtundzwanzigsten Behandlungstag ein modifizierter Schellongtest durchgeführt. Die Registrierung von Blutdruck und Herzfrequenz erfolgte einheitlich immer morgens um dieselbe Uhrzeit unter Verwendung von automatischen Blutdruckregistriergeräten vom Typ Bosomat. Dies sicherte zusätzlich zum Doppelblindverfahren die Objektivität der Blutdruckmessung. Die Messungen wurden im Liegen 15 bzw. 1 Minute vor dem Aufstehen sowie 1, 3, 5, 7 und 9 Minuten nach dem Aufstehen durchgeführt. Vor Erhebung des 1. Messwertes lag in allen Fällen eine mehrstündige Nachtruhe. An den Messtagen erfolgte die morgendliche Einnahme des Präparates unmittelbar nach Ermittlung der Liegewerte 15 Minuten vor dem Aufstehen.

Die statistische Auswertung erfolgte unter Verwendung des Testes nach Dunn-Rankin, des Mann-Whitney-Wilcoxon-Tests und des Friedmann-Tests.

Im Mittelpunkt der objektiven Beurteilung des Behandlungsverlaufs stand der Stehversuch, der einen guten Einblick in die Kreislaufregulationsverhältnisse gewährleistet. Das Verhalten von Blutdruck und Herzfrequenz während der 4-wöchigen Behandlungsphase ist anhand der Werte der sieben Messzeitpunkte (mit Standardabweichung) aus den Tabellen 1 bis 4 ersichtlich. Es handelt sich dabei um die aus dreissig Einzelwerten jeder Patientengruppe errechneten Mittelwerte. Im Rahmen der statistischen Auswertung wurde unter Anwendung des Dunn-Rankin-Tests die Veränderung der Mittelwerte im Liegen, 15 Minuten vor dem Aufstehen, jeweils zwischen zwei aufeinanderfolgenden Messtagen überprüft. Für die Signifikanzprüfung auf Wirkungsunterschiede zwischen den Präparaten Etilefrin und Etilefrinpivalat fand der Mann-Whitney-Wilcoxon-Test Anwendung.

Für die einzelnen Parameter ergab sich folgendes aus den Tabellen ersichtliche Bild:

**Tabelle 1:** Systolischer Blutdruck am Beginn und während der Behandlung mit Etilefrinpivalat und Etilefrin (mm Hg: $\bar{x} \pm s$)

| Kontrolltag / Zeitpunkt | Etilefrinpivalat | | | | Etilefrin | | | |
|---|---|---|---|---|---|---|---|---|
| | vor Beh.-Beginn | 7. Beh.-Tag | 14. Beh.-Tag | 28. Beh.-Tag | vor Beh.-Beginn | 7. Beh.-Tag | 14. Beh.-Tag | 28. Beh.-Tag |
| Liegen/15' vor d. Aufst | 107,3 ± 4,7 | 114,3 ± 6,7 | 119,1 ± 7,7 | 124,7 ± 9,0 | 109,1 ± 6,1 | 112,4 ± 5,7 | 115,2 ± 6,3 | 119,4 ± 6,2 |
| Liegen/ 1' vor d. Aufst | 106,9 ± 4,4 | 114,7 ± 6,4 | 121,1 ± 8,0 | 126,7 ± 9,3 | 108,5 ± 5,8 | 111,3 ± 5,8 | 113,9 ± 6,3 | 118,5 ± 6,6 |
| nach 1' Stehen | 103,7 ± 6,8 | 110,3 ± 7,3 | 116,1 ± 9,0 | 121,7 ±10,5 | 104,2 ± 6,2 | 107,3 ± 6,1 | 110,5 ± 7,5 | 114,6 ± 7,7 |
| nach 3' Stehen | 102,7 ± 6,9 | 109,3 ± 7,8 | 114,1 ± 8,8 | 120,3 ±10,2 | 102,9 ± 6,8 | 106,4 ± 6,4 | 108,9 ± 7,8 | 113,3 ± 8,0 |
| nach 5' Stehen | 101,4 ± 6,9 | 107,6 ± 7,9 | 113,7 ± 8,8 | 119,2 ± 9,7 | 102,3 ± 6,8 | 105,5 ± 6,6 | 107,9 ± 8,0 | 112,5 ± 7,9 |
| nach 7' Stehen | 101,0 ± 6,7 | 107,5 ± 8,0 | 113,7 ± 8,8 | 119,0 ± 9,7 | 101,1 ± 6,6 | 104,1 ± 6,6 | 108,3 ± 8,2 | 112,6 ± 8,3 |
| nach 9' Stehen | 99,7 ± 6,9 | 107,1 ± 7,6 | 113,1 ± 8,1 | 118,8 ± 9,2 | 100,5 ± 6,7 | 103,9 ± 6,3 | 107,3 ± 7,6 | 112,4 ± 7,5 |

0162320

- 7 -  0162320

Wie Tabelle 1 zeigt, nehmen die im Liegen vor der Einnahme von Etilefrin bzw. Etilefrinpivalat erhobenen Messwerte im Verlauf der 4-wöchigen Behandlungsdauer kontinuierlich zu, wobei die zu allen Messtagen erhobenen Werte voneinander signifikant verschieden sind (p < 0,01). So ergibt sich beispielsweise bei Verwendung von Etilefrinpivalat ein Anstieg von 107,3 mm Hg auf 114,3 am 7., 119,1 am 14. und 124,7 mm Hg am 28. Behandlungstag (gemessen jeweils 15 Minuten vor dem Aufstehen) und in der Etilefringruppe von 109,1 mm Hg vor Behandlungsbeginn auf 119,4 mm Hg am 28. Behandlungstag. Ab dem 14. Behandlungstag zeigt sich auch, dass das Etilefrinpivalat hinsichtlich des systolischen Blutdrucks eine grössere Wirksamkeit aufweist als das Etilefrin.

Im Stehen ergibt sich im wesentlichen ein entsprechender Anstieg des systolischen Blutdrucks unter der Einwirkung beider Substanzen. Das bedeutet, dass der Blutdruckeffekt, den die beiden Sympathomimetika in horizontaler Körperlage hervorrufen, im Stehen erhalten bleibt.

In Tabelle 2 sind die Messwerte für den diastolischen Blutdruck dargestellt:

Tabelle 2: Diastolischer Blutdruck am Beginn und während der Behandlung mit Etilefrinpivalat und Etilefrin (mm Hg: $\bar{x} \pm s$)

| Kontrolltag / Zeitpunkt | Etilefrinpivalat | | | | Etilefrin | | | |
|---|---|---|---|---|---|---|---|---|
| | vor Beh.-Beginn | 7. Beh.-Tag | 14. Beh.-Tag | 28. Beh.-Tag | vor Beh.-Beginn | 7. Beh.-Tag | 14. Beh.-Tag | 28. Beh.-Tag |
| Liegen/15' vor d. Aufst | 70,0 ± 3,8 | 71,7 ± 4,4 | 72,5 ± 4,4 | 73,1 ± 3,4 | 69,7 ± 4,9 | 70,7 ± 4,6 | 71,1 ± 3,8 | 72,2 ± 3,6 |
| Liegen/ 1' vor d. Aufst | 69,5 ± 3,6 | 71,5 ± 5,0 | 72,3 ± 4,6 | 72,7 ± 2,9 | 69,1 ± 4,8 | 69,7 ± 4,7 | 70,2 ± 4,0 | 71,3 ± 3,3 |
| nach 1' Stehen | 76,1 ± 6,4 | 77,3 ± 4,8 | 78,8 ± 5,8 | 79,5 ± 4,7 | 75,2 ± 5,4 | 76,1 ± 5,3 | 76,1 ± 5,0 | 76,9 ± 4,4 |
| nach 3' Stehen | 77,9 ± 8,1 | 79,1 ± 5,5 | 79,8 ± 6,1 | 79,8 ± 4,5 | 76,3 ± 5,7 | 77,4 ± 5,9 | 76,7 ± 5,4 | 78,1 ± 4,9 |
| nach 5' Stehen | 77,9 ± 7,2 | 79,9 ± 5,7 | 79,8 ± 5,9 | 79,9 ± 4,9 | 77,3 ± 5,8 | 77,7 ± 5,9 | 77,5 ± 5,7 | 77,5 ± 4,7 |
| nach 7' Stehen | 78,1 ± 7,2 | 79,5 ± 5,9 | 80,1 ± 6,1 | 80,1 ± 4,5 | 76,9 ± 5,8 | 77,5 ± 5,8 | 77,4 ± 5,9 | 78,1 ± 5,4 |
| nach 9' Stehen | 77,6 ± 6,9 | 79,7 ± 5,7 | 80,2 ± 6,0 | 79,5 ± 4,5 | 77,2 ± 6,0 | 77,9 ± 5,7 | 78,0 ± 5,2 | 77,7 ± 4,7 |

6/9

Als wichtiges Ergebnis dieser Tabelle ist festzuhalten, dass beide Wirksubstanzen keine Erhöhung des diastolischen Blutdrucks bedingen und daher keine Anhaltspunkte für eine Steigerung des peripheren Widerstands und somit einer unerwünschten Vasokonstriktion bestehen. Hier ergibt sich im Liegen innerhalb von 28 Behandlungstagen nur ein geringer Anstieg von 70,0 auf 73,1 mm Hg unter Verwendung von Etilefrinpivalat und von 69,7 auf 72,2 mm Hg unter Verwendung von Etilefrin. Entsprechend gering sind auch die Anstiege im Stehen.

Wie aus der nachstehenden Tabelle 3 ersichtlich ist, kommt es entsprechend dem Verhalten von systolischem und diastolischem Blutdruck im Verlauf der Behandlung in beiden Patientengruppen von Messtag zu Messtag zu einer signifikanten Zunahme der Blutdruckamplitude. Sie nimmt unter Etilefrinpivalat von 37,3 mm Hg vor Beginn der Behandlung auf 47,2 mm Hg am 28. Behandlungstag zu und unter Etilefrin von 39,4 mm Hg auf 45,2 mm Hg zu.

Tabelle 3: Blutdruckamplitude am Beginn und während der Behandlung mit Etilefrinpivalat
und Etilefrin (mm Hg: $\bar{x} \pm s$)

| Kontrolltag / Zeitpunkt | Etilefrinpivalat | | | | Etilefrin | | | |
|---|---|---|---|---|---|---|---|---|
| | vor Beh.-Beginn | 7. Beh.-Tag | 14. Beh.-Tag | 28. Beh.-Tag | vor Beh.-Beginn | 7. Beh.-Tag | 14. Beh.-Tag | 28. Beh.-Tag |
| Liegen/15' vor d. Aufst | 37,3 ± 3,8 | 42,5 ± 4,7 | 46,5 ± 6,0 | 47,2 ± 7,0 | 39,4 ± 3,9 | 41,7 ± 3,6 | 44,1 ± 4,9 | 45,2 ± 9,3 |
| Liegen/ 1' vor d. Aufst | 37,3 ± 3,5 | 43,3 ± 5,1 | 48,8 ± 6,4 | 53,9 ± 8,7 | 39,4 ± 3,5 | 41,5 ± 3,8 | 43,7 ± 4,7 | 47,2 ± 6,3 |
| nach 1' Stehen | 27,6 ± 4,4 | 33,1 ± 6,1 | 37,7 ± 7,0 | 42,2 ±10,0 | 29,0 ± 4,9 | 31,2 ± 5,3 | 34,3 ± 6,2 | 37,7 ± 6,4 |
| nach 3' Stehen | 24,8 ± 6,0 | 30,2 ± 5,8 | 34,2 ± 7,4 | 40,5 ± 9,4 | 26,5 ± 5,2 | 29,0 ± 5,5 | 32,2 ± 6,5 | 35,2 ± 7,7 |
| nach 5' Stehen | 23,6 ± 5,9 | 27,7 ± 6,1 | 33,9 ± 6,9 | 39,3 ± 9,0 | 24,9 ± 5,9 | 27,8 ± 5,7 | 30,4 ± 6,8 | 34,9 ± 7,6 |
| nach 7' Stehen | 22,8 ± 6,5 | 28,0 ± 6,5 | 33,7 ± 7,7 | 38,9 ±10,3 | 24,2 ± 5,4 | 26,6 ± 6,0 | 30,9 ± 7,2 | 34,5 ± 8,6 |
| nach 9' Stehen | 22,1 ± 6,8 | 27,3 ± 6,1 | 33,0 ± 7,0 | 39,3 ± 9,1 | 23,3 ± 6,0 | 26,1 ± 6,3 | 29,3 ± 7,0 | 34,7 ± 7,5 |

0162320

**0162320**

Die Ergebnisse gemäss Tabelle 1 und Tabelle 3 zeigen, dass der systolische Blutdruck und die Blutdruckamplitude 15 Minuten vor dem Aufstehen unter der Wirkung beider Substanzen von Messtag zu Messtag ansteigen. Dies bedeutet, da die letzte Einnahme einer Dosis am Mittag des Vortages erfolgte und angesichts der kurzen Halbwertszeit beider Sympathomimetika zum Messzeitpunkt keine wesentliche Substanzkonzentration im Plasma erfolgt, dass langfristig eine deutliche stabile Erhöhung des systolischen Blutdrucks und der Blutdruckamplitude zu verzeichnen ist und somit die therapeutische Wirksamkeit der Sympathomimetika sehr hoch ist.

Wie nachfolgende Tabelle 4 zeigt, bleibt die Herzfrequenz während der Behandlungsdauer bei den gewählten geringen Dosen der Wirkstoffe bei einer erheblichen Erhöhung des Herzschlagvolumens nahezu konstant.

Tabelle 4:   Herzfrequenz am Beginn und während der Behandlung mit Etilefrinpivalat

und Etilefrin (mm Hg: $\bar{x} \pm s$)

| Kontrolltag / Zeitpunkt | Etilefrinpivalat | | | | Etilefrin | | | |
|---|---|---|---|---|---|---|---|---|
| | vor Beh.-Beginn | 7. Beh.-Tag | 14. Beh.-Tag | 28. Beh.-Tag | vor Beh.-Beginn | 7. Beh.-Tag | 14. Beh.-Tag | 28. Beh.-Tag |
| Liegen/15' vor d. Aufst | 71,2 ± 3,4 | 71,5 ± 2,7 | 71,7 ± 2,6 | 71,9 ± 3,0 | 71,1 ± 3,6 | 71,6 ± 3,1 | 71,5 ± 2,9 | 71,3 ± 2,7 |
| Liegen/ 1' vor d. Aufst | 70,4 ± 3,1 | 70,8 ± 2,6 | 70,9 ± 2,9 | 71,3 ± 2,9 | 70,4 ± 3,4 | 70,6 ± 2,9 | 70,6 ± 3,0 | 70,5 ± 2,6 |
| nach 1' Stehen | 87,2 ± 6,7 | 84,5 ± 3,9 | 84,3 ± 4,8 | 83,0 ± 4,1 | 85,8 ± 4,5 | 84,2 ± 5,3 | 83,2 ± 2,9 | 81,5 ± 4,1 |
| nach 3' Stehen | 91,5 ± 5,5 | 87,5 ± 4,3 | 86,8 ± 4,4 | 85,3 ± 4,1 | 89,3 ± 4,7 | 86,8 ± 3,6 | 85,9 ± 4,0 | 83,6 ± 4,0 |
| nach 5' Stehen | 94,2 ± 6,7 | 89,5 ± 4,7 | 88,1 ± 5,1 | 85,5 ± 4,3 | 92,2 ± 5,6 | 88,8 ± 4,5 | 86,7 ± 4,4 | 86,3 ± 4,4 |
| nach 7' Stehen | 94,4 ± 7,5 | 90,4 ± 6,4 | 86,7 ± 5,3 | 85,5 ± 5,0 | 92,2 ± 5,8 | 89,4 ± 6,3 | 87,0 ± 4,5 | 85,3 ± 4,9 |
| nach 9' Stehen | 94,3 ± 8,1 | 90,3 ± 5,6 | 87,4 ± 4,4 | 84,9 ± 9,4 | 91,9 ± 5,5 | 89,2 ± 4,3 | 87,1 ± 3,5 | 84,7 ± 4,6 |

Die verstärkte Wirkung des Etilefrins in Kombination mit Rosskastaniensamenextrakt wird in der nachfolgenden Tabelle 5 gezeigt. Hierzu wurde in einer Doppelblindstudie die Blutdruckwirkung einer Kombination aus Rosskastaniensamenextrakt und Etilefrin (20 mg Etilefrinhydrochlorid und 150 mg Extrakt aus Rosskastaniensamen, standardisiert auf 25 mg Aescin) mit der Wirkung des Etilefrinanteils und von Placebo verglichen. Die anschliessende Behandlung dauerte 4 Wochen.

**Tabelle 5:** Blutddruck und Blutdruckamplitude (mm Hg) während der Stehversuche am Beginn und am Ende der Behandlung

| | Etilefrin u.Roßkastanien-samenextrakt | | Etilefrin retard | | Placebo | |
|---|---|---|---|---|---|---|
| | vor Behandlung | am Ende der Behandlung | vor Behandlung | am Ende der Behandlung | vor Behandlung | am Ende der Behandlung |
| RR syst. im Liegen | 108,0 $\pm$ 5,5 | 123,1 x o $\pm$ 6,7 | 107,3 $\pm$ 4,9 | 118,4 o $\pm$ 7,8 | 106,0 $\pm$ 5,6 | 106,1 $\pm$ 7,1 |
| RR syst. im Stehen | 101,3 $\pm$ 5,9 | 116,6 x o $\pm$ 7,0 | 101,4 $\pm$ 5,3 | 112,4 o $\pm$ 7,8 | 98,9 $\pm$ 4,9 | 99,9 $\pm$ 4,2 |
| RR diast. im Liegen | 68,3 $\pm$ 4,6 | 71,1 o $\pm$ 2,9 | 69,1 $\pm$ 3,9 | 71,0 o $\pm$ 2,7 | 67,0 $\pm$ 4,2 | 66,9 $\pm$ 4,6 |
| RR diast. im Stehen | 75,8 $\pm$ 5,4 | 77,3 $\pm$ 3,1 | 76,8 $\pm$ 3,7 | 78,4 $\pm$ 2,6 | 74,7 $\pm$ 4,3 | 74,8 $\pm$ 4,5 |
| Amplitude im Liegen | 39,7 $\pm$ 4,4 | 52,0 x o $\pm$ 5,9 | 38,3 $\pm$ 5,1 | 47,3 o $\pm$ 6,8 | 39,0 $\pm$ 3,7 | 39,3 $\pm$ 4,4 |
| Amplitude im Stehen | 25,6 $\pm$ 5,8 | 39,3 x o $\pm$ 6,9 | 24,6 $\pm$ 6,6 | 34,0 o $\pm$ 7,6 | 24,2 $\pm$ 3,9 | 25,1 $\pm$ 4,1 |

0162320

Bei Verwendung genannter Kombination ergibt sich eine Steigerung des systolischen Blutdrucks im Liegen um 15,1 mm Hg, während bei Verwendung von Etilefrin eine Steigerung von 11,1 mm Hg zu verzeichnen ist. Bei Gabe von Placebo bleibt der systolische Blutdruck im Liegen mit 106 mm Hg vor und am Ende der Behandlung gleich. Da sich auch bei der Verwendung des Kombinationspräparates bestehend aus Etilefrin und Rosskastaniensamenextrakt der diastolische Blutdruck während der Behandlungsdauer kaum ändert, zeigt auch hier der Anstieg des systolischen Blutdrucks bzw. der Blutdruckamplitude während der Behandlungsdauer die gewünschte Erhöhung des Herzschlagvolumens an.

Die Fig. 1 zeigt den Anstieg des systolischen Blutdruckes bei der 6-monatigen Behandlung und Fig. 2 die dabei beobachtete Änderung der Herzfrequenz.

5029

Klinge Pharma GmbH
EP-59 048

# P a t e n t a n s p r ü c h e

1. Verwendung von Etilefrin oder Etilefrinpivalat zur Herstellung eines Arzneimittels für die Langzeitbehandlung nicht hypotoniebedingter Durchblutungsstörungen.

2. Verwendung von Etilefrin und Etilefrinpivalat nach Anspruch 1 in Kombination mit Roßkastaniensamenextrakt.

112    0162320

Fig. 1 Systolischer (s) und diastolischer (d) Blutdruck
während der 6-monatigen Behandlung mit Etilefrinpivalat (20 mg pro Tag). Nach 6 Monaten wurde die
Behandlung abgebrochen ( ↑ ).
Mittelwerte ± Standardabweichung.

Fig. 2 Herzfrequenz während der 6-monatigen Behandlung
mit Etilefrinpivalat (20 mg pro Tag). Nach 6 Monaten wurde die Behandlung abgebrochen ( ↑ ).
Mittelwerte ± Standardabweichung.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | UNLISTED DRUGS, Band 18, Nr. 12, Dezember 1966, Seite 110, Chatham, New Jersey, US; * Seite 110m: "KD 500" * | 1,2 | A 61 K 31/135 A 61 K 31/70 // (A 61 K 31/70 A 61 K 31:135) |

-----

| RECHERCHIERTE SACHGEBIETE (Int. Cl.4) |
|---|
| A 61 K C 07 C |

Der vorliegende Recherchenbericht wurde für alle Patentansprüche erstellt.

| Recherchenort | Abschlußdatum der Recherche | Prüfer |
|---|---|---|
| DEN HAAG | 23-07-1985 | BRINKMANN C. |